# EUROPEAN PATENT APPLICATION

(11) **EP 3 226 003 A1**
(43) Date of publication of application: **04.10.2017**
(21) Application number: 15863839.5
(22) Date of filing: 27.11.2015
(51) Int. Cl.: G01N 35/08, B01J 19/00, B81B 1/00, G01N 33/543, G01N 37/00, C12M 1/00

(54) **MICRO LIQUID TRANSFER STRUCTURE AND ANALYSIS DEVICE**

(30) Priority: 28.11.2014 JP 2014242226
(71) Applicant: Toyo Seikan Group Holdings, Ltd., Shinagawa-ku Tokyo 141-8627 (JP)
(72) Inventor: OONUKI, Ryuuji, Yokohama-shi Kanagawa 240-0062 (JP); KUNINORI, Masahiro, Yokohama-shi Kanagawa 240-0062 (JP); IWASAKI, Tsutomu, Yokohama-shi Kanagawa 240-0062 (JP)
(74) Representative: Liebetanz, Michael
(86) International application number: PCT/JP2015/005904
(87) International publication number: WO 2016/084381

(57) **Abstract**

Unit rows are periodically arranged in which a plurality of micro projections 60 are arranged in one row and spaces between the adjacent micro projections 60 are formed as liquid transfer paths 61, the micro projections 60 are arranged so that an interval between the respective micro projections 60 becomes an interval to cause a capillary action, and the micro projections 60 are arranged so that in each of the unit rows, at least one of the liquid transfer paths 61 is formed as a low flow resistance liquid transfer path 61 a in which a flow resistance is relatively lowered than in other liquid transfer paths 61 b, and the low flow resistance liquid transfer paths 61 a are disposed along a predetermined liquid transfer direction. Consequently, the liquid transfer direction is optionally controllable with good reproducibility and without causing any deviation in the liquid transfer direction.

## Description

### FIELD

The present invention relates to a micro liquid transfer structure utilizing a capillary action as a propulsive force to transfer a liquid, and more particularly, it relates to a micro liquid transfer structure in which a transfer direction of the liquid is optionally controllable, and an analysis device comprising such a micro liquid transfer structure.

### BACKGROUND

For example, in Patent Literature 1, a micro structure constituting a liquid flow system has been suggested in which a plurality of micro projections are arranged at intervals to cause a capillary action, thereby utilizing the capillary action as a propulsive force for transfer of a liquid.

Furthermore, in Non Patent Literature 1, there is investigated control of fluidity of a liquid in a flow system (a capillary pump) in which such a capillary action is utilized.

Patent Literature 1: Jpn. PCT National Publication No. 2005-532151

Non Patent Literature 1: Capillary pumps for autonomous capillary systems; Lab on a Chip, 2007, 7, 119 to 125

### SUMMARY

### TECHNICAL PROBLEM

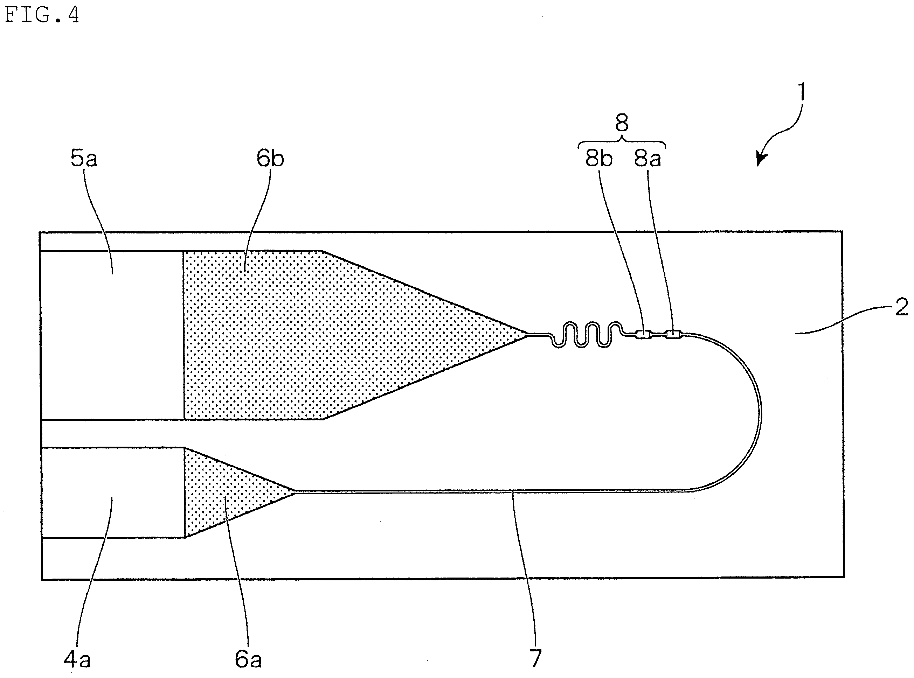

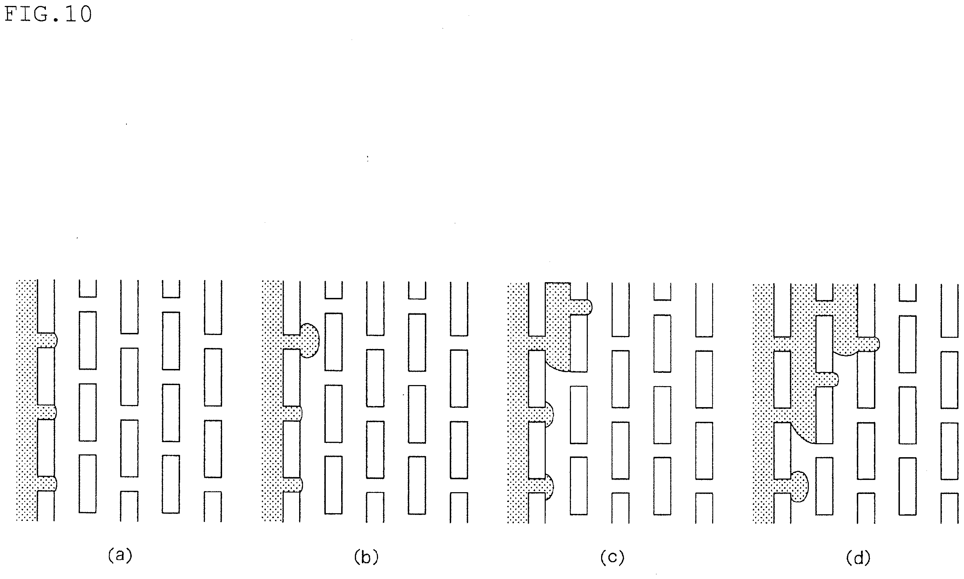

However, according to research by the present inventors, even a flow system of Non Patent Literature 1 is insufficient to control fluidity of a liquid with good reproducibility. For example, FIG. 10 shows one example of a state of the liquid flowing in a capillary pump illustrated in Non Patent Literature 1. In the flow system of Non Patent Literature 1, as shown in FIG. 10, deviation might occur in a liquid transfer direction, and FIG. 4(a) of Non Patent Literature 1 also shows a similar drawing.

The present inventors have earnestly repeated investigation, and in consequence, a reason for the deviation occurrence in the liquid transfer direction may be that, when the liquid flows between respective adjacent micro projections, there is not any difference in flow resistance of the liquid and hence the liquid transfer direction is not determined.

The present invention has been developed in view of the above circumstances, an object thereof is to provide a micro liquid transfer structure which is constituted by arranging a plurality of micro projections at intervals to cause a capillary action, and in the micro liquid transfer structure, a difference is made in flow resistances of liquid transfer paths formed between the micro projections, thereby making it possible to optionally control a liquid transfer direction. Another object thereof is to provide an analysis device comprising such a micro liquid transfer structure.

### SOLUTION TO PROBLEM

A micro liquid transfer structure according to the present invention is directed to a micro liquid transfer structure which comprises a plurality of micro projections arranged at intervals which cause a capillary action and which has a constitution where there are periodically arranged unit rows in which the micro projections are arranged in one row and in which spaces between the adjacent micro projections are formed as liquid transfer paths, and in each of the unit rows, at least one of the liquid transfer paths is formed as a low flow resistance liquid transfer path in which a flow resistance is relatively lowered than in the other liquid transfer paths, and the low flow resistance liquid transfer path is disposed along a predetermined liquid transfer direction.

An analysis device according to the present invention comprises such a micro liquid transfer structure as described above, and has a constitution where a propulsive force is obtained by the micro liquid transfer structure to transfer a prepared liquid including an analysis target.

### ADVANTAGEOUS EFFECTS OF INVENTION

According to the present invention, when a propulsive force of liquid transfer is obtained by a micro liquid transfer structure in which a plurality of micro projections are arranged at intervals to cause a capillary action, a liquid transfer direction is optionally controllable with good reproducibility and without causing any deviation in the liquid transfer direction.

### BRIEF DESCRIPTION OF THE DRAWINGS

FIG. 1 is a perspective view showing one example of an analysis device according to an embodiment of the present invention;
FIG. 2 is an exploded perspective view showing one example of the analysis device according to the embodiment of the present invention;
FIG 3 is an explanatory view of a detecting section in one example of the analysis device according to the embodiment of the present invention;
FIG. 4 is a schematic plan view of a substrate in one example of the analysis device according to the embodiment of the present invention;
FIG. 5 is a schematic plan view showing an enlarged main part of one example of a micro liquid transfer structure according to the embodiment of the present invention;
FIG. 6 is an explanatory view to explain a pinning effect;
FIG. 7 is an explanatory view showing a flowing state of a liquid in one example of the micro liquid transfer structure according to the embodiment of the present invention;
FIG. 8 is an explanatory view showing an example to optionally induce a liquid transfer direction in the example of the micro liquid transfer structure according to the embodiment of the present invention;
FIG. 9 is an explanatory view of another example of the detecting section in one example of the analysis device according to the embodiment of the present invention; and
FIG. 10 is an explanatory view showing a flowing state of a liquid in one example of a conventional micro liquid transfer structure.

### DETAILED DESCRIPTION

Hereinafter, preferable embodiments of the present invention will be described with reference to the drawings.

### [Analysis Device]

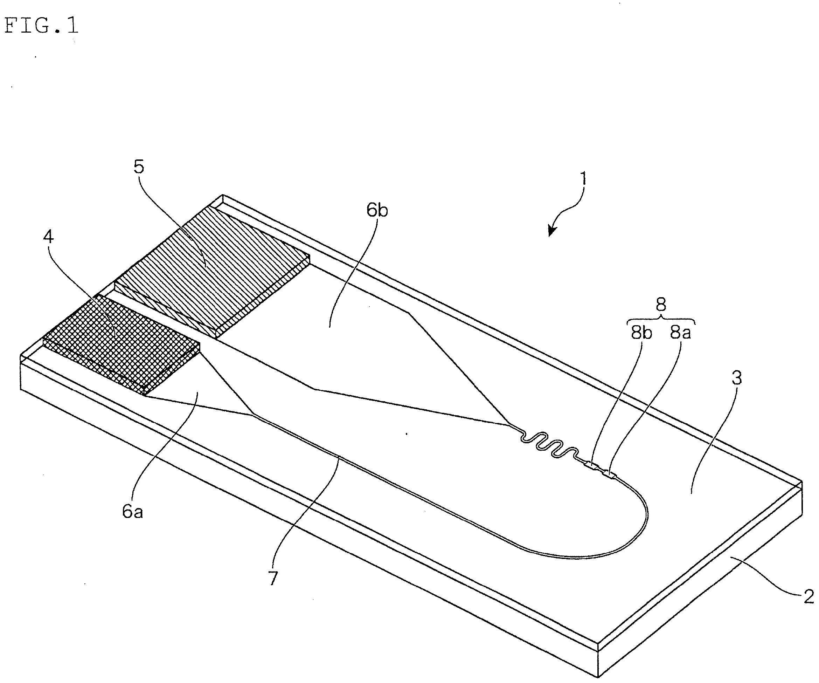

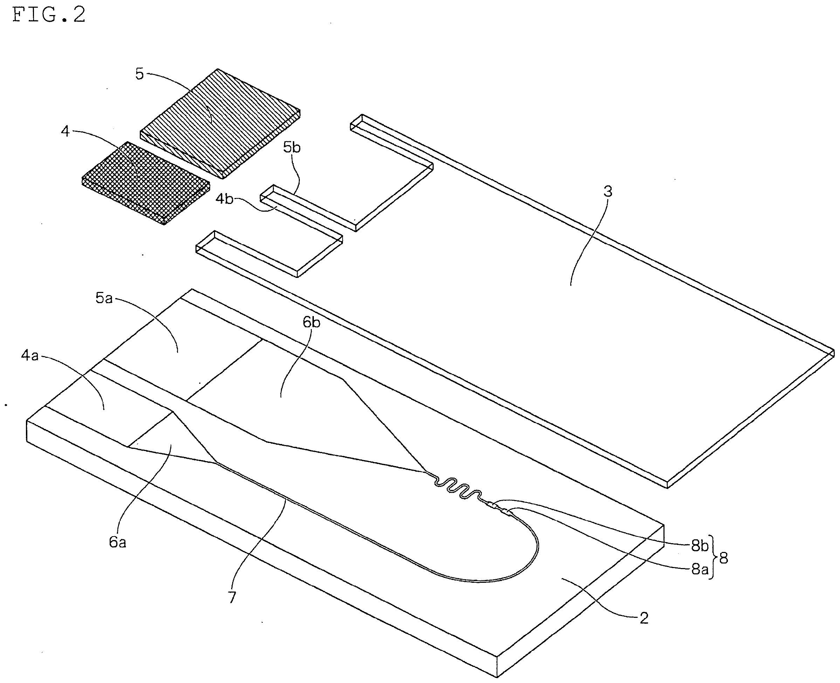

FIG. 1 is a perspective view showing one example of an analysis device according to the present embodiment, and FIG. 2 is an exploded perspective view of the example.

In the example shown in these drawings, an analysis device 1 is constituted as an influenza diagnostic kit by an immunochromatography method, and comprises a substrate 2, a cover 3 that covers the surface of the substrate 2, a conjugate pad 4 impregnated with a gold colloid labelled antibody that combines with an influenza antigen, and an absorbent pad 5 that absorbs a residual liquid after analysis.

In the surface of the substrate 2, there are formed a conjugate pad abutment portion 4a, a first capillary pump portion 6a, a liquid transfer flow path portion 7, a detecting section 8, a second capillary pump portion 6b, and an absorbent pad abutment portion 5a.

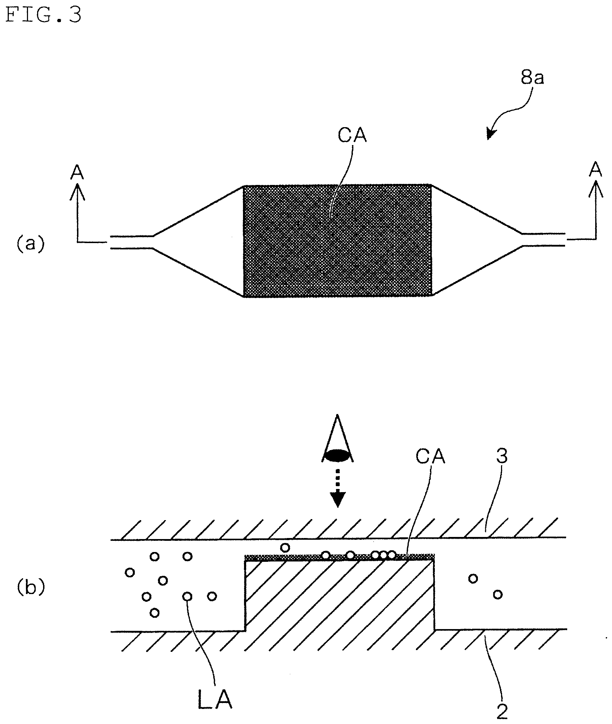

The detecting section 8 is provided with a test flow path 8a coated with a capture antibody that captures the influenza antigen combined with the gold colloid labelled antibody, and a control flow path 8b coated with a capture antibody that captures the gold colloid labelled antibody that is not combined with the influenza antigen. For example, as shown in FIG. 3, the test flow path 8a is formed into a wide and shallow bottom, and its bottom surface is coated with a capture antibody CA, whereby an influenza antigen LA combined with the gold colloid labelled antibody can easily be captured. Although not especially shown in the drawing, the control flow path 8b is also similarly formable.

It is to be noted that FIG. 3(b) is a cross-sectional view along the line A-A in FIG. 3(a).

Furthermore, the first capillary pump portion 6a and the second capillary pump portion 6b are constituted in a micro liquid transfer structure that utilizes a capillary action as a propulsive force to transfer a prepared liquid including an analysis target, and such a micro liquid transfer structure will be described later.

It is to be noted that FIG. 4 is a schematic plan view of the substrate 2, and in the drawing, micro liquid transfer structures are formed into regions shown by halftone dots. On the substrate 2, a micro shape is required to be suitably transferred and formed, and hence the substrate 2 may be molded by subjecting, to cast molding, an ultraviolet curable resin such as polydimethylsiloxane, a thermosetting or a double-fluid curable resin, or may be molded by subjecting, to injection molding, nanoimprint or the like, a thermoplastic resin such as polymethyl methacrylate, a polystyrene, a polycarbonate, a cycloolefin copolymer or a cycloolefin polymer. Alternatively, the substrate may be molded by subjecting a glass or a silicon to etching, ultraprecise machining or the like.

The cover 3 can be made of a resin or a glass, and it is preferable that the cover 3 is transparent to such an extent that the detecting section 8 formed on the surface of the substrate 2 can be seen through the cover 3. The cover 3 is joined to the substrate 2 so as to seal the first capillary pump portion 6a, the liquid transfer flow path portion 7, the detecting section 8 (the test flow path 8a and the control flow path 8b), and the second capillary pump portion 6b, while the conjugate pad 4 and the absorbent pad 5 are held in cutout portions 4b and 5b formed on one end side, respectively.

At this time, the conjugate pad abutment portion 4a is formed to be as deep as or slightly deeper than the bottom surface of the first capillary pump portion 6a so that the first capillary pump portion 6a sealed with the cover 3 opens on the side of the conjugate pad abutment portion 4a. Consequently, via this opening, the conjugate pad 4 is connected to the first capillary pump portion 6a.

Similarly, the absorbent pad abutment portion 5a is formed to be as deep as or slightly deeper than the bottom surface of the second capillary pump portion 6b so that the second capillary pump portion 6b sealed with the cover 3 opens on the side of the absorbent pad abutment portion 5a. Consequently, via this opening, the absorbent pad 5 is connected to the second capillary pump portion 6b.

To diagnose whether or not a test subject is infected with influenza by use of the analysis device 1, a prepared specimen liquid including a specimen (an analysis target) such as nasal mucus sampled from the test subject is first dropped to the conjugate pad 4.

The prepared specimen liquid dropped to the conjugate pad 4 exudes from the conjugate pad 4 and enters the first capillary pump portion 6a. Then, the prepared specimen liquid advances in the first capillary pump portion 6a by the capillary action as the propulsive force, and is transferred to the liquid transfer flow path portion 7. The prepared specimen liquid transferred to the liquid transfer flow path portion 7 flows in the liquid transfer flow path portion 7 by the capillary action, and is transferred to the detecting section 8 formed on the flow path.

A residual liquid of the prepared specimen liquid passed through the detecting section 8 reaches the second capillary pump portion 6b, and advances in the second capillary pump portion 6b by the capillary action as the propulsive force.

At this time, a flow length of the prepared specimen liquid increases, and as a result, a flow resistance also increases in a cumulative manner, so that a liquid transfer speed of the prepared specimen liquid decreases. However, the second capillary pump portion 6b is formed to be widened toward its end as shown in the drawing, and the number of liquid transfer paths 61 formed between micro projections 60 in the micro liquid transfer structure which will be described later increases toward a flow direction, thereby making it possible to inhibit the decrease of the liquid transfer speed due to the flow resistance. Consequently, the prepared specimen liquid can be transferred at a constant flow rate without using any external pump, thereby enabling analysis (diagnosis) with high reproducibility.

The residual liquid of the prepared specimen liquid advances in the second capillary pump portion 6b and is then absorbed in the absorbent pad 5.

When the prepared specimen liquid is dropped to the conjugate pad 4, the gold colloid labelled antibody impregnated into the conjugate pad 4 dissolves in the prepared specimen liquid. Then, if a patient is infected with influenza, the influenza antigen is contained in the prepared specimen liquid. Consequently, a part of the gold colloid labelled antibody dissolved in the prepared specimen liquid combines with the influenza antigen, and is transferred to the detecting section 8 formed on the flow path of the liquid transfer flow path portion 7, together with the residual gold colloid labelled antibody that is not combined with the influenza antigen.

As described above, the detecting section 8 is provided with the test flow path 8a coated with the capture antibody that captures the influenza antigen combined with the gold colloid labelled antibody, and the control flow path 8b coated with the capture antibody that captures the gold colloid labelled antibody that is not combined with the influenza antigen. Therefore, when the prepared specimen liquid is passed through the detecting section 8 and then a color generated by gold colloidal particles is visually recognized only in the control flow path 8b, it can be diagnosed that the test subject is not infected with influenza. On the other hand, when the color generated by the gold colloidal particles are visually recognized also in the test flow path 8a, it can be diagnosed that the test subject is infected with influenza.

### [Micro Liquid Transfer Structure]

Next, there will be described the micro liquid transfer structure in which the capillary action is utilized as the propulsive force of the liquid transfer to transfer the prepared liquid including the analysis target in the analysis device 1 mentioned above.

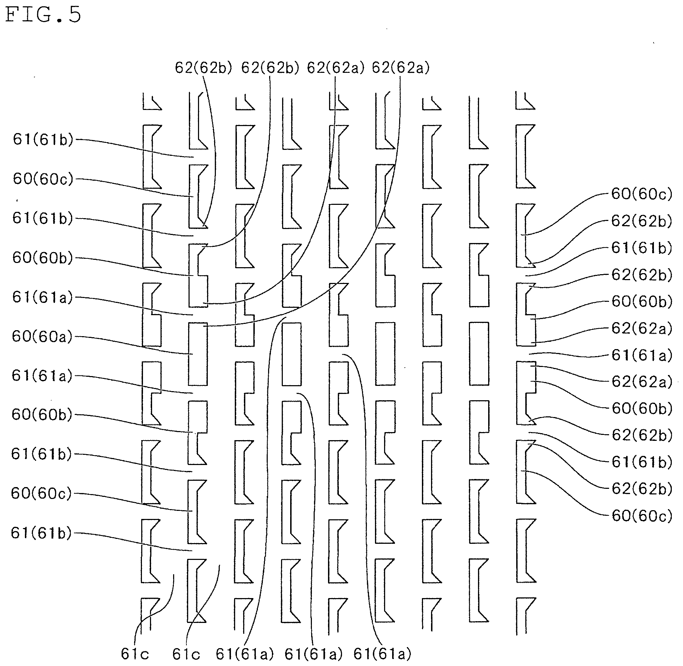

FIG. 5 is a schematic plan view showing an enlarged main part of one example of the micro liquid transfer structure according to the present embodiment. In such a micro liquid transfer structure, there are periodically arranged unit rows in which a plurality of micro projections 60 are arranged in one row and in which each space between adjacent micro projections 60 is formed as the liquid transfer path 61, and the unit rows are arranged so that each of intervals between the micro projections 60 is an interval to cause the capillary action (e.g., the interval between the adjacent micro projections 60 in one unit row is from 1 to 1000 µm and an interval between adjacent unit rows is from 1 to 1000 µm).

It is to be noted that in the example shown in FIG. 5, the micro projection 60 has a longitudinal size of 120 µm, a lateral size of 30 µm, and a height of 30 µm, the interval between the adjacent micro projections 60 in one unit row is 30 µm, and the interval between the adjacent unit rows is 60 µm. In the analysis device 1 mentioned above, the height of the micro projection 60 corresponds to a depth of the bottom surface of each of the first capillary pump portion 6a and the second capillary pump portion 6b which are constituted of the micro liquid transfer structures according to the present embodiment, and tips of the micro projections 60 come in contact closely with the cover 3. Consequently, a space around the micro projections 60 is sealed by the cover 3.

Furthermore, in the present embodiment, the micro projections 60 are uniformly arranged so that the liquid transfer paths 61 formed into one unit row and the liquid transfer paths 61 formed into a unit row adjacent to this one unit row are positioned alternately as shown in the drawing. Consequently, when the liquid passes through the liquid transfer paths 61 formed into the one unit row by the capillary action and the liquid surface comes in contact with the micro projections 60 of the adjacent unit row, the liquid spreads between these unit rows by the capillary action, and then these are repeated. Then, due to the above arrangement, the liquid transfer paths of the liquid by the capillary action are formed as in a parallel circuit, and the flow resistance can be decreased as compared with a case where the liquid transfer paths are formed as in a series circuit, so that the propulsive force of the liquid transfer in which the capillary action is utilized can efficiently be obtained.

Furthermore, in the example shown in FIG. 5, on the micro projections 60 which are adjacent to form the liquid transfer path 61, edge portions 62 which develop a pinning effect are formed on an outlet side of each of the liquid transfer paths 61.

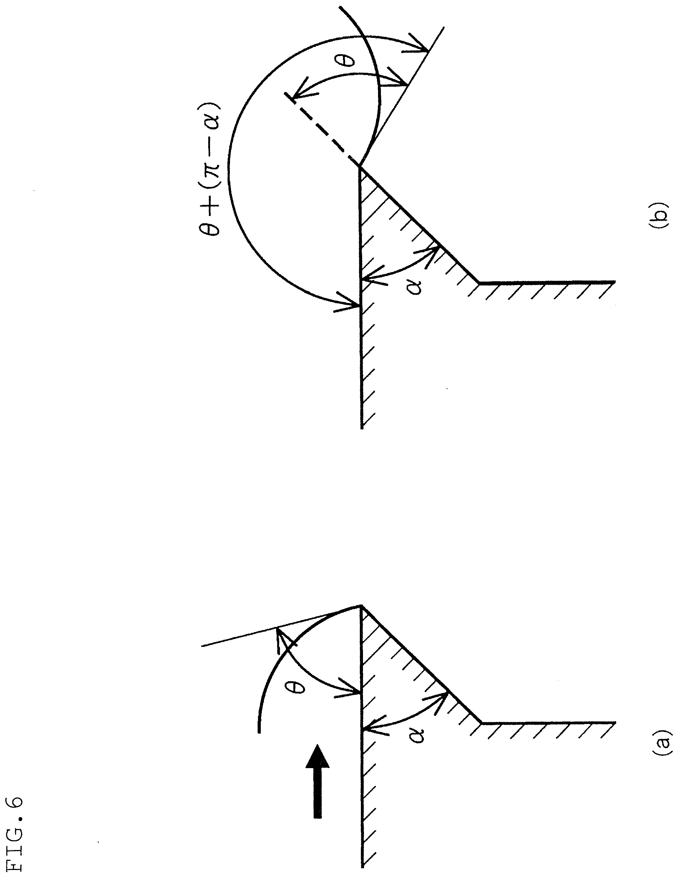

Here, as shown in FIG. 6, the pinning effect is a phenomenon where when the liquid surface advanced along a plane at a contact angle θ reaches an edge portion (see FIG 6(a)), the liquid surface does not ride over the edge portion until the contact angle reaches θ+(π-α) in which α is an angle formed by the plane and an outer surface of the edge portion (see FIG 6(b)), and in the present embodiment, the angle α formed by the plane and the outer surface of the edge portion at this time is defined as a pinning angle.

In the example shown in FIG. 5, by suitably setting the pinning angle of the edge portion 62 formed on the outlet side of the liquid transfer path 61, at least one of the liquid transfer paths 61 formed between the micro projections 60 in the one unit row is formed as a low flow resistance liquid transfer path 61 a in which the flow resistance is relatively lowered than in the other liquid transfer paths 61 b.

That is, the pinning angle of an edge portion 62b formed on the outlet side of the liquid transfer path 61 b of adjacent micro projections 60b and 60c which form the other liquid transfer paths 61 b exclusive of the low flow resistance liquid transfer path 61 a is decreased than the pinning angle of an edge portion 62a formed on the outlet side of the low flow resistance liquid transfer path 61 a of adjacent micro projections 60a and 60b which form the low flow resistance liquid transfer path 61 a, whereby the flow resistance of the low flow resistance liquid transfer path 61 a is relatively lowered than flow resistances of the other liquid transfer paths 61 b.

It is to be noted that in the example shown in FIG. 5, the micro projection 60a is formed into a rectangular shape and the edge portions 62a having a pinning angle of 90° are formed at both ends of the micro projection. Furthermore, in the micro projection 60b, the edge portion 62a having a pinning angle of 90° is formed at one end, and the edge portion 62b having a pinning angle of 45° is formed at the other end, and in the micro projection 60c, the edge portions 62b having a pinning angle of 45° are formed at both ends. Tips of the edge portions 62a and 62b may be rounded to such an extent that development of the pinning effect is not obstructed.

Furthermore, in the example shown in FIG. 5, in each unit row, the micro projections 60a, 60b and 60c are arranged so that at least one of the liquid transfer paths 61 is formed as the low flow resistance liquid transfer path 61 a in which the flow resistance is relatively lowered than in the other liquid transfer paths 61 b and so that the low flow resistance liquid transfer path 61 a is disposed in a predetermined liquid transfer direction. Furthermore, for example, by suitably adjusting the interval between the adjacent unit rows (e.g., by increasing the interval in consideration of an interval between the adjacent micro projection 60a and 60b which form the low flow resistance liquid transfer path 61 a), a flow resistance of an inter-row liquid transfer path 61 c formed between the unit rows is relatively lowered than the flow resistance of the low flow resistance liquid transfer path 61 a.

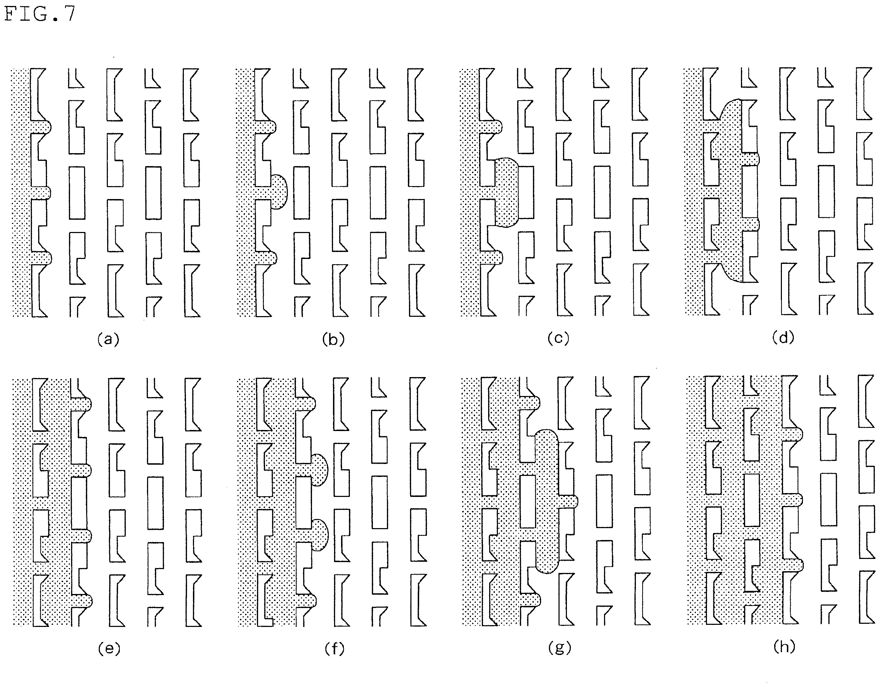

Thus, as shown in FIG. 7, the liquid preferentially passes through the low flow resistance liquid transfer path 61 a having the low flow resistance among the liquid transfer paths 61 formed into one unit row (see FIGS. 7(a) to (c)), and this is a trigger for the capillary action, so that the liquid spreads into the inter-row liquid transfer path 61 c formed between the unit row and the next unit row (see FIGS. 7(c) to (e)), but since the flow resistance of the inter-row liquid transfer path 61 c is lowest, a tip of the liquid does not advance to the inter-row liquid transfer path 61 c formed between the next unit row and a unit row next to the next unit row until the inter-row liquid transfer path 61 c is filled with the liquid (see FIG. 7(e)). Then, when the inter-row liquid transfer path 61c is filled with the liquid, the liquid preferentially passes through the low flow resistance liquid transfer paths 61 a of the next unit row (see FIG. 7(f)), and by the repeat of this behavior, the liquid flows (see FIGS. 7(f) to (h)).

Consequently, according to the present embodiment, the capillary action can be utilized to transfer the liquid, so that it is possible to suitably control fluidity with good reproducibility and without causing any deviation in the liquid transfer direction.

Furthermore, in the analysis device 1 mentioned above, each of the first capillary pump portion 6a and the second capillary pump portion 6b is formed into an isosceles triangular shape, and the low flow resistance liquid transfer portion 61 a is arranged along a perpendicular line drawn from a vertex of the triangle to a base thereof, whereby the propulsive force of the liquid transfer by utilizing the capillary action in the micro liquid transfer structure can efficiently be obtained. However, the arrangement of the low flow resistance liquid transfer portion 61 a can suitably be set in accordance with a desirable liquid transfer direction.

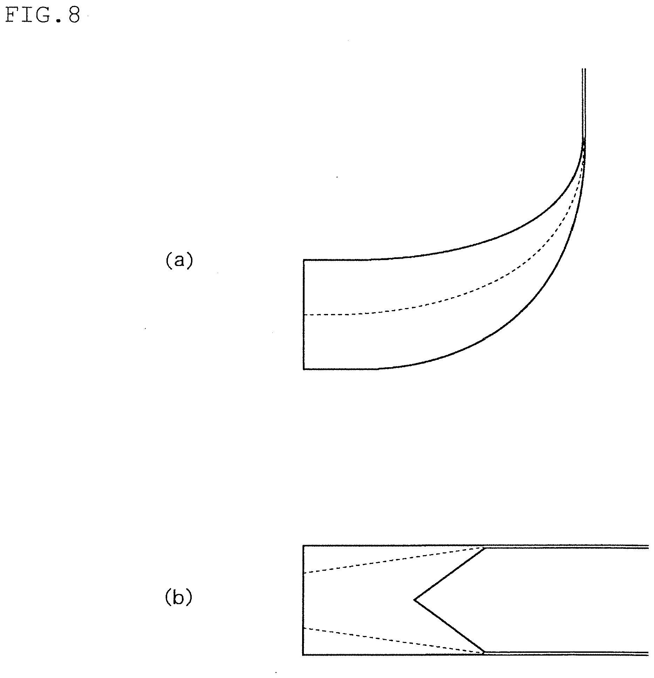

For example, in a case where it is desired to curve the liquid transfer direction as shown in FIG. 8(a) or a case where it is desired to branch the liquid transfer direction into two or more directions as shown in FIG. 8(b), the low flow resistance liquid transfer path 61 a is disposed along a direction shown by a dotted line in the drawing, thereby making it possible to induce the liquid transfer direction in an optional direction.

Thus, according to the present embodiment, in obtaining the propulsive force of the liquid transfer by the micro liquid transfer structure comprising the plurality of micro projections arranged at the intervals which causes the capillary action, the liquid transfer direction can optionally be controlled with good reproducibility and without causing any deviation in the liquid transfer direction.

In the above, the present invention has been described with reference to the preferable embodiments, but needless to say, the present invention is not limited to the above-mentioned embodiments, and various changes and operations can be carried out in the scope of the present invention.

For example, in the above-mentioned embodiment concerned with the micro liquid transfer structure, for the purpose of relatively lowering the flow resistance of the low flow resistance liquid transfer path 61 a than the flow resistances of the other liquid transfer paths 61 b, the edge portions 62 which develop the pinning effect are formed on the outlet side of the liquid transfer path 61 of the adjacent micro projections 60 which form the liquid transfer path 61, and the pinning angle of the edge portion 62 is suitably set, thereby relatively decreasing the flow resistance of the low flow resistance liquid transfer path 61 a than the flow resistances of the other liquid transfer paths 61 b, but the present invention is not limited to this embodiment.

The flow resistance of the low flow resistance liquid transfer path 61 a may relatively be lowered than the flow resistances of the other liquid transfer paths 61 b, for example, as follows.

1) The edge portions 62 which develop the pinning effect are formed on the outlet side of the liquid transfer paths 61 b only in the adjacent micro projections 60 which form the other liquid transfer paths 61 b, and for example, a shape on the outlet side of the liquid transfer path 61 a of the adjacent micro projections which form the low flow resistance liquid transfer path 61 a is adjusted into a rounded shape that does not develop any pinning effect, thereby relatively lowering the flow resistance of the low flow resistance liquid transfer path 61 a than the flow resistances of the other liquid transfer paths 61 b.
2) A region on the other liquid transfer path 61 b side of the adjacent micro projections 60 which form the other liquid transfer paths 61 b is subjected to a hydrophobic treatment to increase the flow resistance, thereby relatively lowering the flow resistance of the low flow resistance liquid transfer path 61 a than the flow resistances of the other liquid transfer paths 61 b.
3) A sectional area of the low flow resistance liquid transfer path 61 a is increased, thereby relatively lowering the flow resistance of the low flow resistance liquid transfer path 61 a than the flow resistance of the other liquid transfer path 61 b.

Furthermore, in the above-mentioned embodiment, there has been described the example of suitably adjusting the intervals between the adjacent unit rows for the purpose of relatively lowering the flow resistance of the inter-row liquid transfer path 61 c formed between the unit rows than the flow resistance of the low flow resistance liquid transfer path 61 a, but the present invention is not limited to this embodiment. By use of a technique shown in each of the above 1) to 3) or the like, the flow resistance of the inter-row liquid transfer path 61 c may relatively be lowered than the flow resistance of the low flow resistance liquid transfer path 61 a.

Furthermore, the shape of the micro projection 60 is not limited to that of the above-mentioned embodiment, and can suitably be designed in such a range that does not obstruct the effect of the present invention.

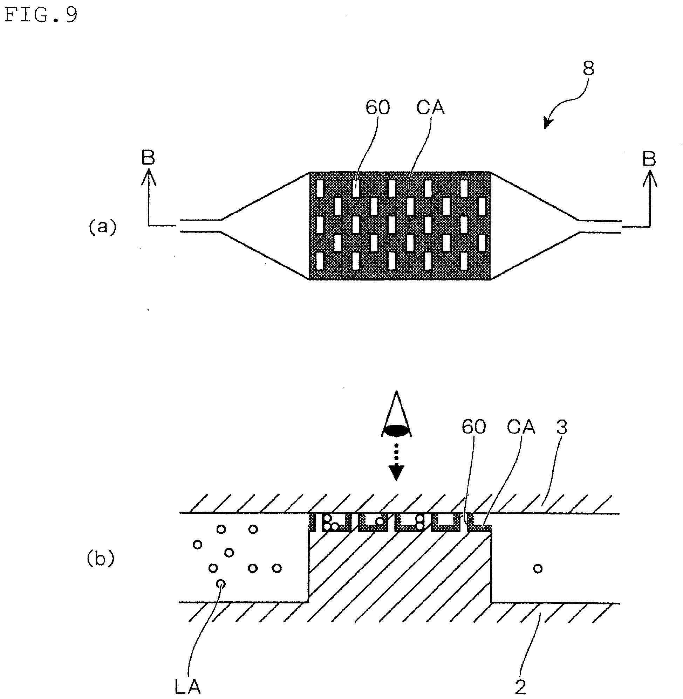

Additionally, in the above-mentioned embodiment concerned with the analysis device, there has been described the example where the test flow path 8a and the control flow path 8b provided in the detecting section 8 are formed into the wide and shallow bottom, and their bottom surfaces are coated with the capture antibody, but the present invention is not limited to this embodiment. As shown in FIG. 9, such micro liquid transfer structures as described above may be formed also in the test flow path 8a and the control flow path 8b provided in the detecting section 8, thereby controlling the liquid transfer direction of the prepared specimen liquid passing through these flow paths 8a and 8b. At this time, the micro projections 60 may also be coated with the capture antibody CA, thereby making it easier to capture the influenza antigen LA combined with the gold colloid labelled antibody, for example, in the test flow path 8a, and the influenza antigen LA combined with the gold colloid labelled antibody is captured also in a height direction of the micro projections 60, thereby making it easier to visually recognize the color developed by the gold colloidal particles.

It is to be noted that FIG. 9(b) is a cross-sectional view along the line B-B of FIG 9(a).

Furthermore, in the above-mentioned embodiment concerned with the analysis device, the influenza diagnostic kit has been described as one example, but the present invention is not limited to this embodiment. The present invention is applicable to various analysis devices in each of which the capillary action is utilized for acquirement of the propulsive force to transfer a prepared liquid including an analysis target without using any external pump as liquid transfer means.

### EXAMPLES

Next, the present invention will be described in more detail with reference to specific examples.

### [Example]

In a mold prepared by performing micro processing in accordance with a photolithography method, polydimethylsiloxane (SILPOT184 manufactured by Dow Corning Toray and having a weight ratio of 10:1 to a hardener) was cast, and a substrate was formed so that a capillary pump (a micro liquid transfer structure) of the same arrangement as in an example shown in FIG. 5 was transferred and formed on the surface of the substrate. A cover made of large size slide glass (manufactured by Matsunami Glass Ind., Ltd.) was brought into contact closely with the surface of the substrate obtained in this way by self-adsorption of polydimethylsiloxane to seal the capillary pump.

### [Comparative Example 1]

The procedure of Example 1 was repeated except that a capillary pump having all micro projections arranged in the same manner as in micro projections 60a of an example shown in FIG. 5 was transferred and formed on a substrate surface.

### [Comparative Example 2]

The procedure of Example 1 was repeated except that a capillary pump having all micro projections arranged in the same manner as in micro projections 60c of an example shown in FIG 5 was transferred and formed on a substrate surface.

### [Evaluation]

As a prepared liquid, 3% TRITON-X100 (manufactured by The Dow Chemical Company) was used, and dropped at a drop rate of 5 µL into an input port provided in a cover. The prepared liquid flowing in a capillary pump by a capillary force was photographed with a digital microscope (VHK-1000 manufactured by Keyence Corporation), and a time for which the prepared liquid passed through the capillary pump was measured, whereby a variability of the flow rate (coefficient of variation; CV) was calculate. The measurement was carried out five times, and Table 1 shows the average value. Table 1 also shows that evaluation of a micro liquid transfer structure having a CV of 5% or less is represented by "○", which indicates the good structure capable of stably transferring liquid, and shows that evaluation of a micro liquid transfer structure having a CV in excess of 5% is represented by "×".

**[Table 1]**

| | Example | Comparative Example① | Comparative Example② |
|---|---|---|---|
| Capillary pump detailed view | 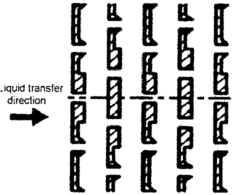 | 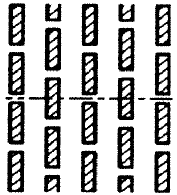 | 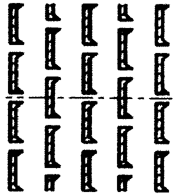 |
| Low flow resistance liquid transfer path Pinning angle | 90° | None | None |
| Liquid transfer path Pinning angle | 45° | 90° | 45° |
| Variability of flow rate (CV) | 3.5% | 22.5% | 11.8% |
| General evaluation | ○ | × | × |

### INDUSTRIAL APPLICABILITY

The present invention is applicable and utilizable not only to analysis devices in medical fields as influenza diagnostic kits and others but also to analysis devices in various fields.

There are herein referred to all contents of the literatures described in this description and the Japanese application based on the Paris convention.

### REFERENCE SIGNS LIST

1 analysis device
60 (60a, 60b and 60c) micro projections
61 liquid transfer path
61 a low flow resistance liquid transfer path
61 b another liquid transfer path
61 c inter-row liquid transfer path
62 (62a and 62b) edge portion

## Claims

1. A micro liquid transfer structure which comprises a plurality of micro projections arranged at intervals which cause a capillary action,
wherein there are periodically arranged unit rows in which the micro projections are arranged in one row and a space between the adjacent micro projections is formed as a liquid transfer path,
in each of the unit rows, at least one of the liquid transfer paths is formed as a low flow resistance liquid transfer path in which a flow resistance is relatively lowered than in the other liquid transfer paths, and
the low flow resistance liquid transfer path is disposed along a predetermined liquid transfer direction.

2. The micro liquid transfer structure according to claim 1,
wherein a flow resistance of an inter-row liquid transfer path formed between the unit rows is relatively lowered than a flow resistance of the low flow resistance liquid transfer path.

3. The micro liquid transfer structure according to claim 1 or 2,
wherein an edge portion which develops a pinning effect is formed on an outlet side of a liquid transfer path of the adjacent micro projections which form the other liquid transfer paths exclusive of the low flow resistance liquid transfer path,
whereby the flow resistance of the low flow resistance liquid transfer path is relatively lowered than flow resistances of the other liquid transfer paths.

4. The micro liquid transfer structure according to claim 1 or 2,
wherein an edge portion which develops a pinning effect is formed on an outlet side of the liquid transfer path of the adjacent micro projections which form the liquid transfer path, and
a pinning angle of the edge portions formed on the adjacent micro projections which form the other liquid transfer paths exclusive of the low flow resistance liquid transfer path is decreased than a pinning angle of the edge portions formed on the adjacent micro projections which form the low flow resistance liquid transfer path,
whereby the flow resistance of the low flow resistance liquid transfer path is relatively lowered than the flow resistances of the other liquid transfer paths.

5. The micro liquid transfer structure according to claim 1 or 2,
wherein a region on the side of the liquid transfer path of the adjacent micro projections which form the other liquid transfer paths exclusive of the low flow resistance liquid transfer path is subjected to a hydrophobic treatment,
whereby the flow resistance of the low flow resistance liquid transfer path is relatively lowered than the flow resistances of the other liquid transfer paths.

6. The micro liquid transfer structure according to claim 1 or 2,
wherein a sectional area of the low flow resistance liquid transfer path is increased than sectional areas of other liquid transfer paths exclusive of the low flow resistance liquid transfer path,
whereby the flow resistance of the low flow resistance liquid transfer path is relatively lowered than flow resistances of the other liquid transfer paths.

7. An analysis device which comprises the micro liquid transfer structure according to any one of claims 1 to 5 and in which a propulsive force is obtained by the micro liquid transfer structure to transfer a prepared liquid including an analysis target.
